(19) Europäisches Patentamt
European Patent Office
Office européen des brevets

(11) **EP 3 109 336 B1**

(12) **EUROPEAN PATENT SPECIFICATION**

(45) Date of publication and mention
of the grant of the patent:
**14.08.2019 Bulletin 2019/33**

(51) Int Cl.:
**C22C 45/10** $^{(2006.01)}$     **A61C 8/00** $^{(2006.01)}$
**A61K 6/04** $^{(2006.01)}$     **A61K 6/00** $^{(2006.01)}$

(21) Application number: **15752219.4**

(22) Date of filing: **05.02.2015**

(86) International application number:
**PCT/JP2015/053303**

(87) International publication number:
**WO 2015/125625 (27.08.2015 Gazette 2015/34)**

(54) **DENTAL MEMBER**

DENTALELEMENT

ÉLÉMENT DENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.02.2014 JP 2014032174**

(43) Date of publication of application:
**28.12.2016 Bulletin 2016/52**

(73) Proprietors:
• **Maruemu Works Co., Ltd
Osaka-shi, Osaka 542-0086 (JP)**
• **Tohoku Techno Arch Co., Ltd.
Sendai-shi, Miyagi 980-0845 (JP)**

(72) Inventors:
• **Yamamoto Teruko
Sendai-shi
Miyagi 980-8577 (JP)**
• **Yokoyama Yoshihiko
Sendai-shi
Miyagi 980-8577 (JP)**

(74) Representative: **Plougmann Vingtoft a/s
Strandvejen 70
2900 Hellerup (DK)**

(56) References cited:
WO-A1-2013/008870    JP-A- S6 440 058
JP-A- H07 289 567    JP-A- H08 131 460
JP-A- 2004 097 787    JP-A- 2004 097 787
JP-A- 2009 137 888    JP-A- 2009 215 610
JP-A- 2009 215 610    US-A- 5 324 368
US-A1- 2004 072 124

• **TARIQ N H ET AL: "Effect of prior compression
treatment on the deformation behavior of Zr
based bulk metallic glass", MATERIALS
CHEMISTRY AND PHYSICS, vol. 143, no. 3, 14
February 2014 (2014-02-14), pages 1384-1390,
XP028807663, ISSN: 0254-0584, DOI:
10.1016/J.MATCHEMPHYS.2013.11.050**
• **ASAHI KAWASHIMA ET AL: "The corrosion
behaviour of Zr-based bulk metallic glasses in
0.5M NaCl solution", CORROSION SCIENCE,
OXFORD, GB, vol. 53, no. 9, 5 May 2011
(2011-05-05), pages 2778-2784, XP028230026,
ISSN: 0010-938X, DOI:
10.1016/J.CORSCI.2011.05.014 [retrieved on
2011-05-10]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

The present invention relates to a dental member.

Description of the Related Art

In recent years, dental implants are frequently used, which are embedded into the jaw bone to replace missing tooth roots after tooth loss. Orthodontic anchor screws are also frequently used in various cases of malocclusion as anchorage sources for the movement of teeth under orthodontic treatment. The use of orthodontic anchor screws as anchorage sources for orthodontic treatment enables precise and efficient movement of teeth, which has been conventionally difficult, regardless of the degree of patient cooperation. As dental members including such dental implants and orthodontic anchor screws, dental members made of pure titanium or titanium alloy having relatively high strength and good biocompatibility have been conventionally used.

In addition, metallic glass is known as a material having properties such as high strength, low Young's modulus, and high corrosion resistance. As a highly ductile metallic glass alloy that is excellent in plastic workability and is applicable to metal working process such as cold press working, a metallic glass alloy having a composition represented by formula: $Zr_aNi_bCu_cAl_d$ [wherein a, b, c, and d denote at.%, "a" ranges from 60 to 75 at.%, "b" ranges from 1 to 30 at. %, "c" ranges from 1 to 30 at.%, and "d" ranges from 5 to 20 at.%] has been proposed by the present inventors (for example, see Patent Literature 1).

Other previously proposed arrangements are disclosed in TARIQ N H ET AL: "Effect of prior compression treatment on the deformation behavior of Zr based bulk metallic glass", MATERIALS CHEMISTRY AND PHYSICS, vol. 143, no. 3, pages 1384-1390, 14 February 2014, in ASAHI KAWASHIMA ET AL: "The corrosion behaviour of Zr-based bulk metallic glasses in 0.5M NaCl solution", CORROSION SCIENCE, vol. 53, no. 9, pages 2778-2784, 5 May 2011, in JP2009215610A and in US2004072124A1.

PRIOR ART LITERATURE

Patent Literature

Patent Literature 1: JP Patent Publication (Kokai) No. 2009-215610 A

SUMMARY OF THE INVENTION

Conventional titanium dental members are relatively large or long with respect to the installation positions, and thus are problematic in that they can have a risk of damaging adjacent tooth roots upon implantation. Further, these dental members are also problematic in that they can be broken or fractured in use. Moreover, these dental members can become unstable or fall off during orthodontic treatment, and thus are problematic in that they are unstable unless used for mature bone.

The present invention has been achieved noting these problems. An object of the present invention is to provide a dental member, which has a reduced size, and thus can prevent damages to tooth roots upon implantation, has higher strength and lower elasticity compared with titanium dental members, and is excellent in stability of engraftment to bone after implantation.

In order to achieve the above object, the dental member according to the present invention is characterized by containing an amorphous alloy having a composition represented by formula: $Zr_aNi_bCu_cAl_d$ wherein a, b, c, and d denote at.%, "a" ranges from 67 to 73 at.%, "b" ranges from 11 to 17 at.%, "c" ranges from 5 to 13 at.%, and "d" ranges from 5 to 9 at.%.

The dental member according to the present invention contains an amorphous alloy having higher strength and lower elasticity compared with titanium dental members such as titanium alloy or pure titanium dental members, so that it is hard to be broken or fractured in use, and has a low risk of damaging adjacent tooth roots. Furthermore, the dental member according to the present invention accelerates the formation of new bone around the member after implantation unlike titanium dental members, so that the dental member of the present invention is excellent in stability of engraftment to bone after implantation, and has a low risk of becoming unstable or falling off during orthodontic treatment.

The dental member according to the present invention may be any member such as a bridge, an orthodontic bracket, an orthodontic wire, and an orthodontic band, as long as it is a member that can be formed with an amorphous alloy. In particular, the dental member of the present invention preferably comprises an orthodontic anchor screw or a dental implant.

In general, a titanium orthodontic anchor screw has a length of 6-8 mm and a diameter (outer diameter) of 1.4 mm or more, and a titanium dental implant has a length of 8-12 mm and a diameter of 3 mm or more. However, the dental member according to the present invention has higher strength and lower elasticity compared with titanium dental members and is excellent in stability of engraftment to bone, so that the dental member of the present invention can be formed shorter and thinner compared with titanium dental members. For example, the dental member of the present invention can be an orthodontic anchor screw wherein the screw part has a core diameter between 0.5 mm and 1.0 mm or a length between 2 mm and 5 mm, a one-piece-type dental implant wherein the screw part has the largest diameter between 0.5 and 2.9 mm and a length between 2 and 13.4 mm, or, a two-piece-type dental implant wherein the screw part has the largest diameter between 0.5 and 2.9 mm and a length between 2 and 5.9 mm. The dental member of the present invention is shorter and thinner than titanium dental members, but can exert strength, elasticity, and an effect of new bone generation equivalent to or better than those of titanium dental members. Moreover, size reduction can prevent damages to tooth roots upon implantation.

The dental member according to the present invention is characterized in that "a" ranges from 67 to 73 at.%, "b" ranges from 11 to 17 at.%, "c" ranges from 5 to 13 at.%, and "d" ranges from 5 to 9 at.%. In this case, the dental member of the present invention has particularly high strength and low elasticity, and is excellent in stability of engraftment to bone after implantation. Furthermore, preferably, "c" ranges from 7 to 13 at. %, or, "d" ranges from 5 to 7 at.%. Furthermore, "a" may range from 69 to 73 at.%, "b" may range from 13 to 17 at.%, "c" may range from 5 to 10 at.%, and "d" may range from 5 to 9 at. %.

The dental member has preferably the surface coated with zirconia ceramics. In this case, the dental member can be produced by heating the above amorphous alloy in an atmosphere in which oxygen is present at temperatures where no crystallization or no embrittlement takes place. In a specific example, the dental member can be produced by heating the amorphous alloy in air at temperatures ranging from 350°C to 400°C. Since the surface of the amorphous alloy is coated with very strong zirconia ceramics, leading to improved strength. Moreover, zirconia ceramics on the surface can prevent internal nickel from dissolving and affecting the human body.

According to the present invention, size reduction of the dental member can prevent damages to tooth roots upon implantation, and thus the dental member having higher strength and lower elasticity compared with titanium dental members and being excellent in stability of engraftment to bone after implantation can be provided.

BRIEF DESCRIPTION OF THE DRAWINGS

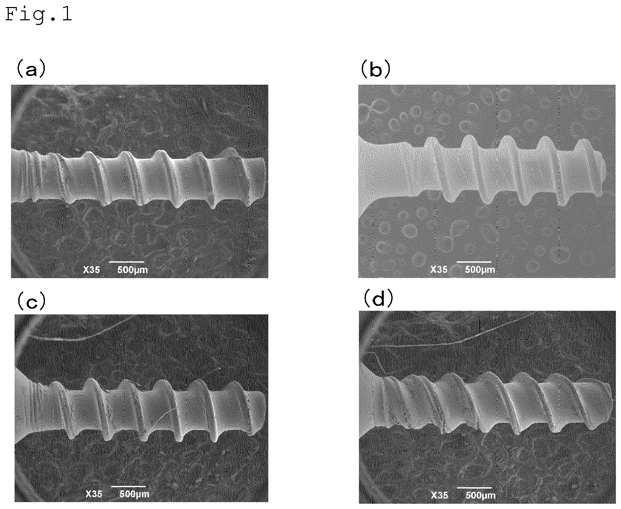

Fig. 1 shows scanning electron microscope (SEM) photograms (a) a pure titanium screw, as well as the dental members of embodiments of the present invention, orthodontic anchor screws made of amorphous alloys having compositions (b) $Zr_{70}Ni_{16}Cu_6Al_8$, (c) $Zr_{68}Ni_{12}Cu_{12}Al_8$, and (d) $Zr_{72}Ni_{16}Cu_6Al_6$, used in experiments.

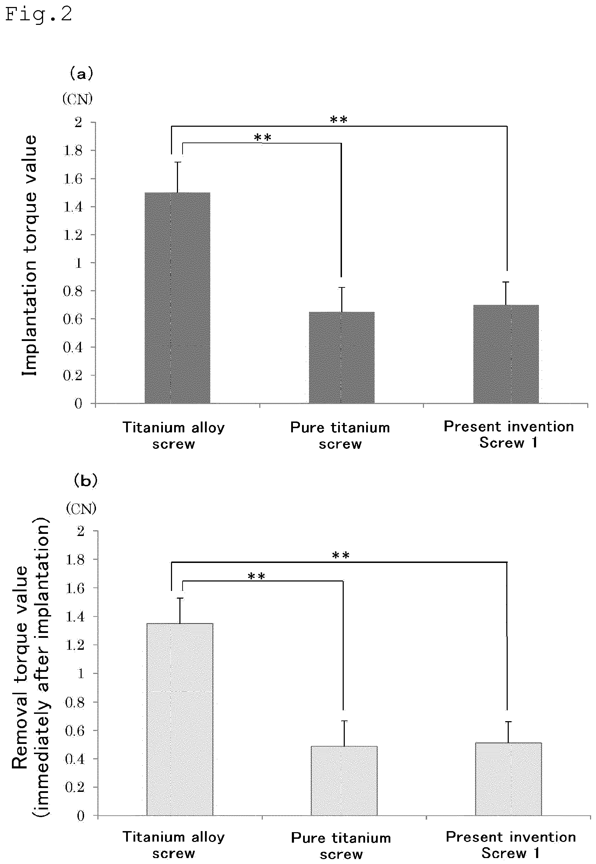

Fig. 2 shows graphs (a) implantation torques and (b) removal torques immediately after implantation in a test of implantation of the dental member of an embodiment of the present invention, an orthodontic anchor screw made of an amorphous alloy having a composition of $Zr_{70}Ni_{16}Cu_6Al_8$, into rat tibia.

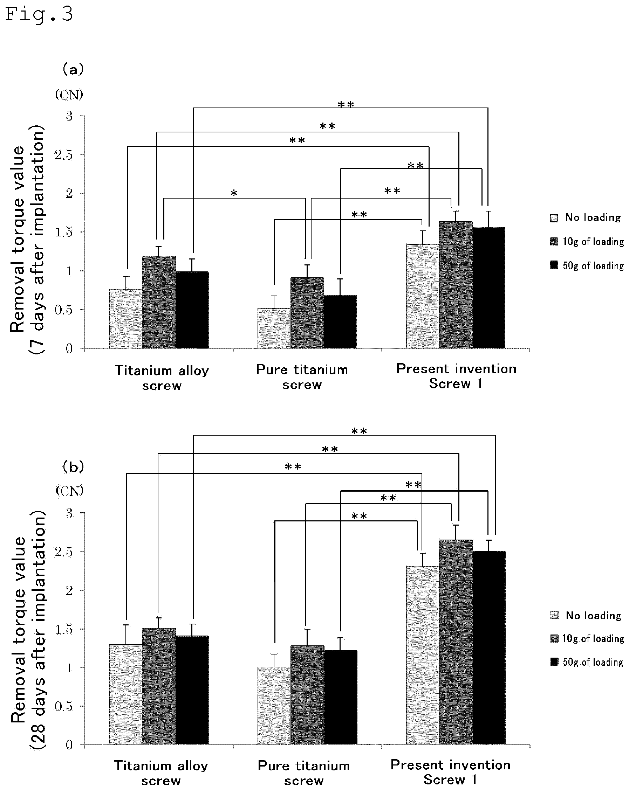

Fig. 3 shows graphs (a) removal torques seven days after implantation and (b) removal torques 28 days after implantation in a test of implantation of the dental member of an embodiment of the present invention into rat tibia.

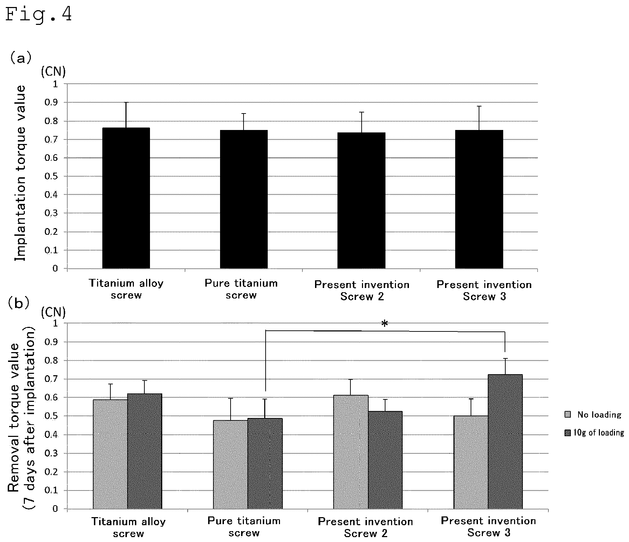

Fig. 4 shows graphs (a) implantation torques and (b) removal torques seven days after implantation in a test of implantation of the dental members of embodiments of the present invention, orthodontic anchor screws made of amorphous alloys having compositions of $Zr_{68}Ni_{12}Cu_{12}Al_8$ and $Zr_{72}Ni_{16}Cu_6Al_6$, into rat tibia.

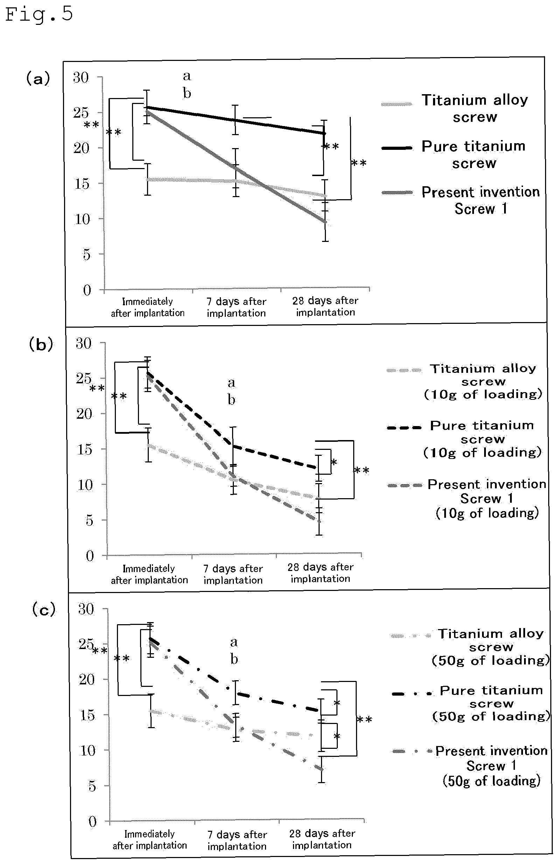

Fig. 5 shows graphs showing changes over time in mobility after implantation (Periotest value) in a test of implantation of the dental member of an embodiment of the present invention, an orthodontic anchor screw made of an amorphous alloy having a composition of $Zr_{70}Ni_{16}Cu_6Al_8$ subjected to (a) no immediate loading, (b) 10g of immediate loading, and (c) 50 g of immediate loading into rat tibia.

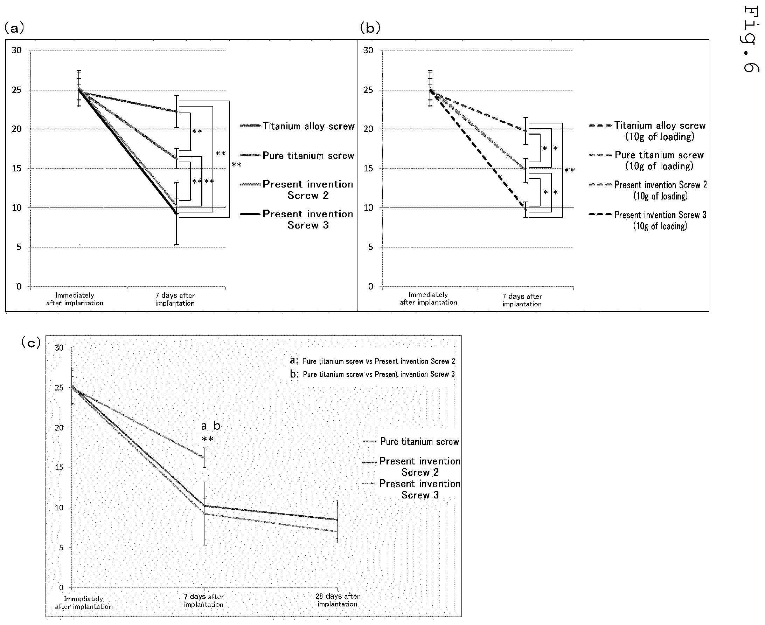

Fig. 6 shows graphs showing changes over time in mobility after implantation (Periotest value) in a test of implantation of the dental members of embodiments of the present invention, orthodontic anchor screws made of amorphous alloys having compositions $Zr_{68}Ni_{12}Cu_{12}Al_8$ and $Zr_{72}Ni_{16}Cu_6Al_6$ subjected to (a) no immediate loading until seven days after implantation, (b) 10 g of immediate loading until seven days after implantation, and (c) no immediate loading until 28 days after implantation into rat tibia.

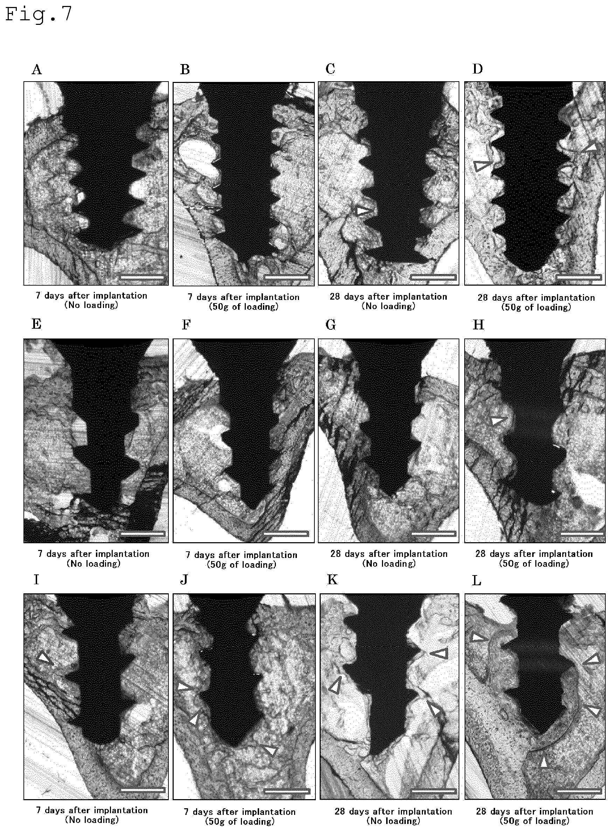

Fig. 7 shows optical microscopic photographs (Scale bars; 1.0 mm) showing undecalcified tissue sections of tibia seven and 28 days after implantation, in a test of implantation of a titanium alloy screw (A) to (D), a pure titanium screw (E) to (H), and the dental member of an embodiment of the present invention (I) to (L); that is, an orthodontic anchor screw made of an amorphous alloy having a composition of $Zr_{70}Ni_{16}Cu_6Al_8$, into rat tibia.

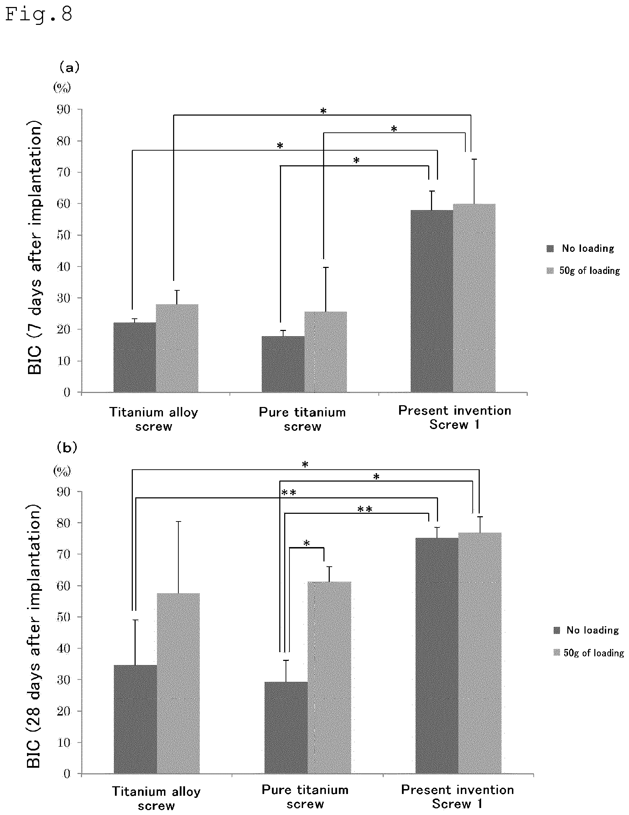

Fig. 8 shows graphs (a) BIC seven days after implantation and (b) BIC 28 days after implantation of each screw found on the basis of optical microscopic photographs shown in Fig. 7.

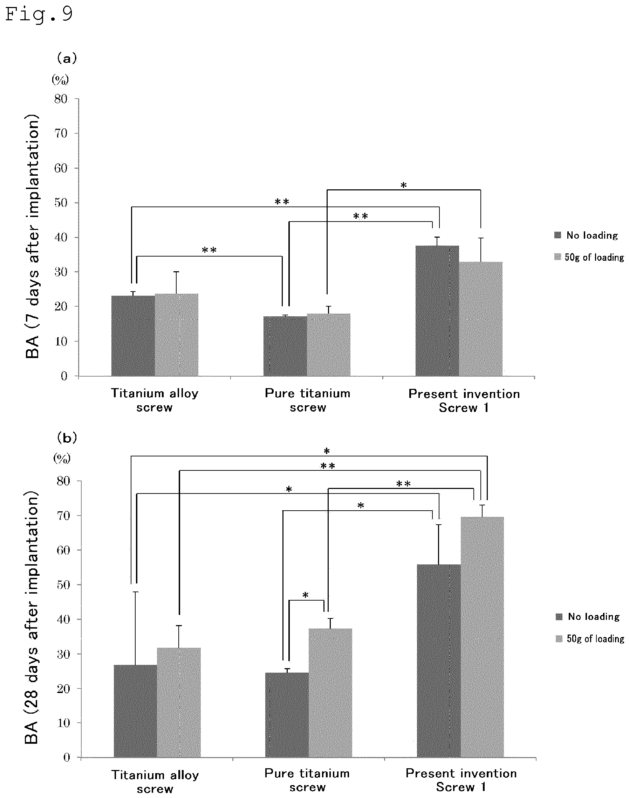

Fig. 9 shows graphs (a) BA seven days after implantation and (b) BA 28 days after implantation of each screw found on the basis of the optical microscopic photographs shown in Fig. 7.

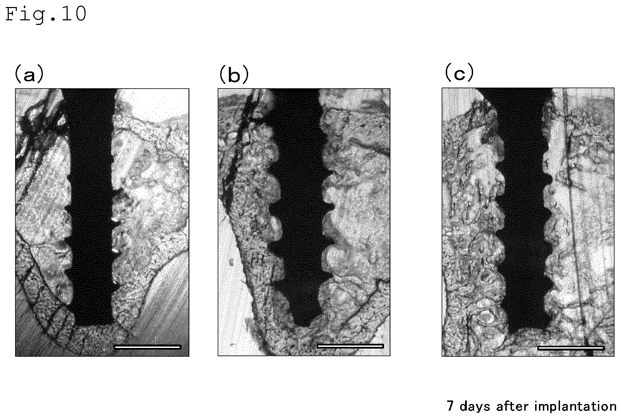

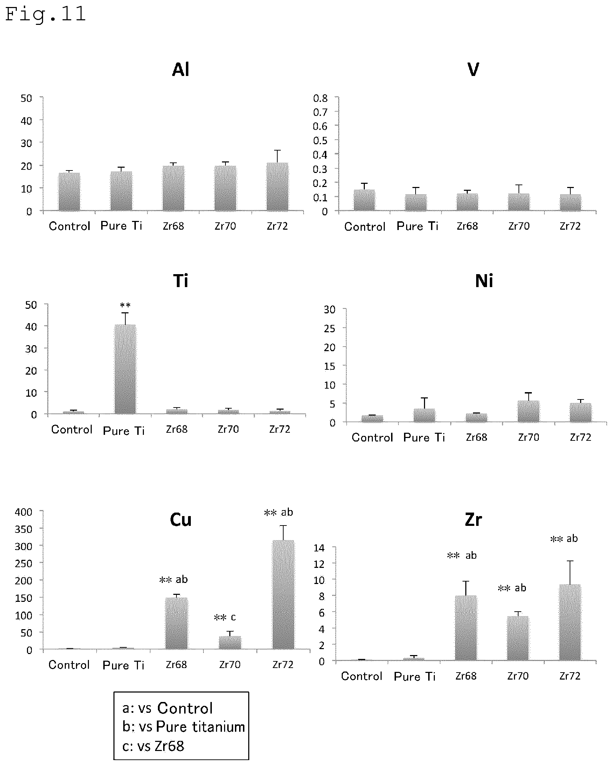

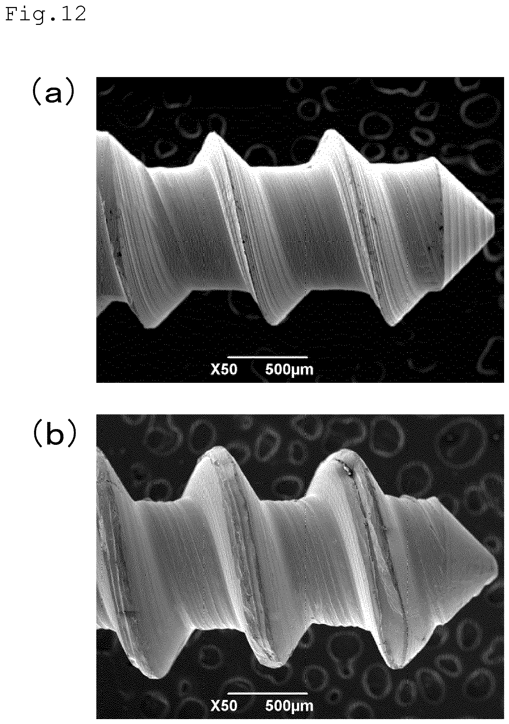

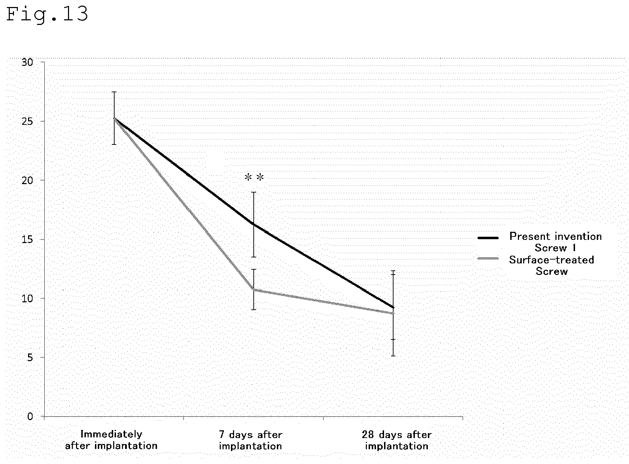

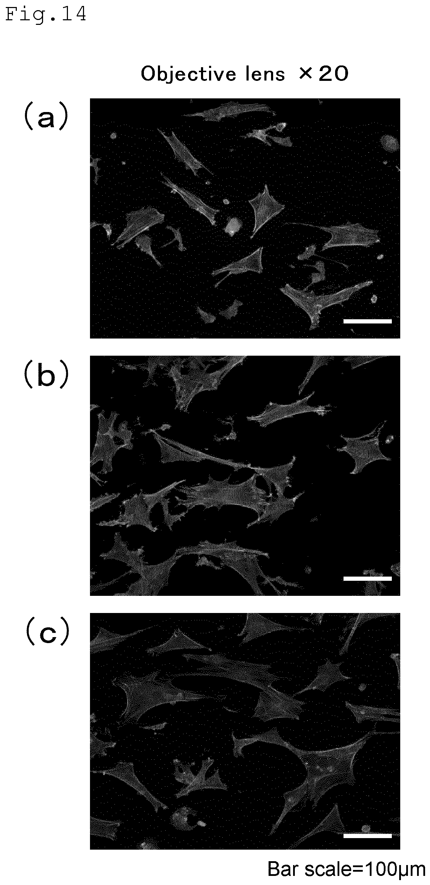

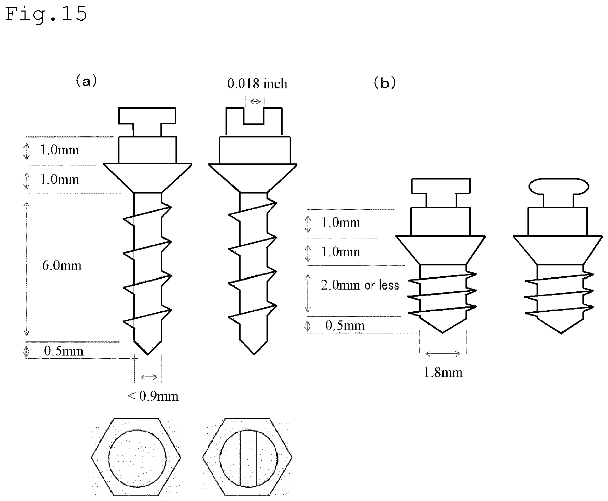

Fig. 10 shows optical microscopic photographs (Scale bars; 1.0 mm) showing undecalcified tissue sections of tibia seven days after implantation, in a test of implantation of (a) a pure titanium screw, as well as the dental members of embodiments of the present invention, orthodontic anchor screws made of amorphous alloys having compositions of (b) $Zr_{68}Ni_{12}Cu_{12}Al_8$ and (c) $Zr_{72}Ni_{16}Cu_6Al_6$, into rat tibia.Fig. 11 shows graphs (vertical axis; dissolution amount [ppt]) showing the results of testing the dissolution of each metal component of $Zr_{70}Ni_{16}Cu_6Al_8$ (Zr70), $Zr_{68}Ni_{12}Cu_{12}Al_8$ (Zr68) and $Zr_{72}Ni_{16}Cu_6Al_6$ (Zr72) amorphous alloys composing the dental members of embodiments of the present invention, orthodontic anchor screws and pure titanium (pure Ti) composing a pure titanium screw.Fig. 12 shows scanning electron microscope (SEM) photographs showing the dental member of an embodiment of the present invention, (a) an orthodontic anchor screw made of an amorphous alloy having a composition of $Zr_{70}Ni_{16}Cu_6Al_8$, and (b) an orthodontic anchor screw made of a surface-treated $Zr_{70}Ni_{16}Cu_6Al_8$ amorphous alloy having the surface coated with zirconia ceramics, which were used in an experiment.Fig. 13 is a graph showing changes over time in mobility after implantation (Periotest value) in a test of implantation of the dental member of an embodiment of the present invention, an orthodontic anchor screw made of an amorphous alloy having a composition of $Zr_{70}Ni_{16}Cu_6Al_8$, and, an orthodontic anchor screw made of a surface-treated $Zr_{70}Ni_{16}Cu_6Al_8$ amorphous alloy having the surface coated with zirconia ceramics, into rat tibia.Fig. 14 shows confocal microscope photographs showing the results of observing the surfaces of (a) a pure titanium foil, (b) a $Zr_{70}Ni_{16}Cu_6Al_8$ amorphous alloy foil used for the dental member of an embodiment of the present invention, and (c) a surface-treated $Zr_{70}Ni_{16}Cu_6Al_8$ amorphous alloy foil after one day of culture in a cell adhesion test.Fig. 15 shows (a) a front view, a side view, a bottom view, and a plan view of an example of the screw shape, and (b) a front view and a side view of an example of the button shape, which are the dental members of embodiments of the present invention.

## DESCRIPTION OF THE PREFERRED EMBODIMENTS OF THE INVENTION

The embodiments of the present invention will be described in detail as follows with reference to the drawings. The dental member of the present invention comprises an amorphous alloy having a composition represented by formula: $Zr_aNi_bCu_cAl_d$ wherein a, b, c, and d denote at.%, "a" ranges from 67 to 73 at.%, "b" ranges from 11 to 17 at.%, "c" ranges from 5 to 13 at.%, and "d" ranges from 5 to 9 at.%.

This amorphous alloy can be produced by metal mold casting, for example. Specifically, first, raw materials including zirconium (Zr), nickel (Ni), copper (Cu), and aluminium (Al) are weighed and mixed to result in a desired composition, and then the mixture is melted and mixed in an inert gas atmosphere to generate a mother alloy. Next, the mother alloy is melted again in air, and then casting is performed with a copper template by an arc-melting tilt-casting method or the like, so that the amorphous alloy can be produced. The shaped raw material of the thus produced amorphous alloy is subjected to machining, and then the dental member of an embodiment of the present invention can be produced.

The dental member of an embodiment of the present invention is made of an amorphous alloy with higher strength and lower elasticity compared with titanium dental members such as titanium alloy dental members and pure titanium dental members, so that the dental member is hard to be broken or fractured in use and has a low risk of damaging adjacent tooth roots. Moreover, the dental member of an embodiment of the present invention accelerates the formation of new bone around the member after implantation unlike titanium dental members. Hence, the dental member of the present invention is excellent in stability of engraftment to bone after implantation, and has a low risk of becoming unstable or falling off during orthodontic treatment.

In addition, an amorphous alloy shaped in a desired shape is heated in air at 350°C to 400°C to oxidize the surface, and thus a dental member having the surface coated with zirconia ceramics can be produced. In this case, the surface of an amorphous alloy is covered with very strong zirconia ceramics, so that the strength can be increased.

Experiments shown below were conducted to verify the effect of the dental member of an embodiment of the present invention.

Examples

[Experimental outline]

As the dental members of embodiments of the present invention, an orthodontic anchor screw made of an amorphous alloy having a composition of $Zr_{70}Ni_{16}Cu_6Al_8$ (hereinafter, referred to as "screw 1 of the present invention"),

an orthodontic anchor screw made of an amorphous alloy having a composition of $Zr_{68}Ni_{12}Cu_{12}Al_8$ (hereinafter, referred to as "screw 2 of the present invention"), and an orthodontic anchor screw made of an amorphous alloy having a composition of $Zr_{72}Ni_{16}Cu_6Al_6$ (hereinafter referred to as "screw 3 of the present invention") were produced and subjected to an experiment using rats. Screws 1 to 3 of the present invention used in this experiment are shown in Fig. 1(b) to (d), respectively. In addition, the surfaces of screws 1 to 3 of the present invention were not coated with zirconia ceramics.

As shown in Fig. 1(b) to (d), each of the produced orthodontic anchor screws had a diameter (outer diameter) of 1.3 mm, a length of 3.0 mm, and a pitch of 0.8 mm. In addition, as comparative samples, a pure titanium screw (99.4% titanium, pitch: 0.8 mm), and a titanium alloy screw (pitch: 0.5 mm), which had the same shape as the other screws, were produced and subjected to the same experiment. The pure titanium screw used in this experiment is shown in Fig. 1(a). The titanium alloy screw used herein was AbsoAnchor (Dentos) that is widely used today in clinical settings.

Rats used in this experiment were 12-week-old male Wistar rats (body weight: 250 g to 260 g). To reduce the burden on animals due to the experiment, the procedure was performed by intraperitoneal injection of 5 mg/ml pentobarbital under general anesthesia.

Moreover, in an experiment for measuring values, the thus obtained experimental data were statistically processed by performing one-way analysis of variance, and then performing a multiple comparison test by Tukey-Kramer method. In this test, a significant difference was determined with the significance level of 1% ($P<0.01$) or 5% ($P<0.05$), a result with a significance level of less than 1% is denoted as "**" and a result with a significance level of less than 5% is denoted as "*" in each drawing.

[Implantation of screws and application of immediate loading]

Medial surfaces of both lower extremities of each of a plurality of rats were shaved, skin and fascia incisions, each having an about 15.0 mm long, were made in parallel to the long axis of rat tibia, and then the medial surfaces of the tibiae were exposed with raspatories. When immediate loading was applied, drilling was performed at two positions per a side (a position in the vicinity of and a position in the central part of the joint at the boundary with the femur) at a low rotational speed using a round bar, so as to form a 1.0-mm fossa for implantation of a screw. When no immediate loading was applied, drilling was performed at one position per side (in the vicinity of the joint at the boundary with the femur), so as to form a 1.0-mm fossa for implantation of a screw.

When immediate loading was applied, a screw was implanted with an anchor driver, vertically with respect to the cortical bone at an implantation part. Skin and fascial incisions were sutured using nonabsorbable sutures (Keisei Medical Industrial Co., Ltd. "SU-1160NS"). The surgical fields were sterilized with iodine cotton balls, and then 10 g or 50 g of immediate loading was applied by screw types to each screw using coil springs (TOMY INTERNATIONAL INC. "SENTALLOY coil springs") made of an orthodontic nickel titanium alloy.

Loading was applied immediately after implantation of each screw. After implantation of each screw, loading was kept applied for seven and 28 days of evaluation. In addition, screws to be evaluated were screws implanted in the vicinity of the joint at the boundary with the femur, from among screws implanted in tibiae. Screws implanted in the central parts of tibiae were used as anchorage sources when immediate loading was applied. As the screws for anchorage sources, AbsoAnchor processed to have a length of 3.0 mm was used consistently.

Moreover, when no immediate loading was applied, each screw was implanted with an anchor driver vertically with respect to cortical bone at an implantation part, skin and fascial incisions were sutured with nonabsorbable sutures (Keisei Medical Industrial Co., Ltd. "SU-1160NS"), and then the surgical fields were sterilized with iodine cotton balls. After implantation of each screw, it was retained for seven or 28 days of evaluation.

[Implantation of screws and measurement of removal torque values]

Screw 1 of the present invention, a titanium alloy screw, and a pure titanium screw were measured for implantation torque values and removal torque values immediately after, seven days after implantation, and 28 days after implantation, respectively, of each screw using a torque gauge (Tohnichi). Implantation torque values were measured by screw types of four screws implanted in the joints. Furthermore, removal torque values were measured by screw types of four screws immediately after implantation. Removal torque values were also measured by screw types of four screws and by weights of immediate loading seven and 28 days after implantation. The highest torque value among torque values of each screw type was employed as a measured torque value. The results of measuring implantation torque values and removal torque values immediately after implantation are shown in Fig. 2(a) and (b), respectively. Moreover, the results of measuring removal torque values seven days after implantation and removal torque values 28 days after implantation are shown in Fig. 3(a) and (b), respectively.

As shown in Fig. 2(a), the implantation torque of screw 1 of the present invention was 0.7N cm, that of the titanium alloy screw was 1.5N cm, and that of the pure titanium screw was 0.65N cm. It was thus confirmed that the implantation torque value of the titanium alloy screw was significantly higher than those of the other two screw types.

Furthermore, as shown in Fig. 2(b), the removal torque immediately after implantation of screw 1 of the present invention was 0.5 IN cm, that of the titanium alloy screw was 1.35N cm, and that of the pure titanium screw was 0.49N cm. It was thus confirmed that the removal torque immediately after implantation of the titanium alloy screw was significantly higher than those of the other two screw types, exhibiting the same tendency as in the case of implantation torques. This may be because the pitch width (0.5 mm) of the titanium alloy screw was shorter than the pitch width (0.8 mm) of the other two screw types.

Furthermore, as shown in Fig. 2, it was confirmed for all screws that the removal torque value immediately after implantation was lower than the implantation torque value. This may be because the bone wall of a fossa for implantation was damaged at the time of implantation of each screw, so that the contact area between the screw and bone was decreased from that at the time of implantation.

As shown in Fig. 3, it was confirmed for all screws that the removal torque value 28 days after implantation was significantly higher than the same seven days after implantation. This may be because new bone was formed around each screw as time proceeded after implantation. In addition, it was also confirmed for all screws that the removal torque seven days after implantation and the same 28 days after implantation were found to be highest when 10g of immediate loading was applied.

As shown in Fig. 3, the removal torque seven days after implantation and the same 28 days after implantation of screw 1 of the present invention were confirmed to be significantly higher than the other two screw types in all immediate loading groups. This may be because more new bone sites were formed around screw 1 of the present invention than those formed around the other two screw types. As shown in Fig. 2 and Fig. 3, the removal torque value of the screw 1 of the present invention increased from the torque immediately after implantation more significantly than those of the other two screw types, suggesting that more new bone sites were quickly formed than those formed around the other two screw types. Accordingly, it was considered that screw 1 of the present invention after implantation became stable more quickly than the other two screw types, maintaining good stability.

Next, screw 2 and screw 3 of the present invention were similarly measured for implantation torque values and removal torque values seven days after implantation. In addition, for comparison, a titanium alloy screw and a pure titanium screw were also measured. The results of measuring implantation torque values and removal torque values seven days after implantation are shown in Fig. 4(a) and (b), respectively.

As shown in Fig. 4(a), it was confirmed for all screws that implantation torque values were almost the same. Moreover, as shown in Fig. 4(b), the removal torque values seven days after implantation were confirmed to be lower than the implantation torque values, except for screw 3 of the present invention subjected to 10g of immediate loading. This may be because even seven days after implantation, the bone wall of a fossa for implantation, which had been damaged at the time of implantation of a screw, remained affected.

Moreover, as shown in Fig. 4(b), the removal torque value seven days after implantation of screw 3 of the present invention subjected to 10g of immediate loading was higher than those of the other screws, and was particularly confirmed to be significantly higher than that of the pure titanium screw subjected to 10g of immediate loading. This may be because more new bone sites were formed around screw 3 of the present invention subjected to 10g of immediate loading, than those formed around the other screws.

[Measurement of screw mobility]

For measurement of the stability of the screws after implantation, Screw 1 of the present invention, a titanium alloy screw, and a pure titanium screw were measured for the mobility of the screws implanted into tibiae immediately after implantation, seven days after implantation, and 28 days after implantation using a mobility measuring device, Periotest (Gulden Messtechnik). Measurement was performed by applying Periotest vertically with respect to each screw head portion at 3 sites (one site in the longitudinal direction of tibiae, two sites resulting from 120° rotation from the first site), the mean value of these 3 values was designated as a measurement value (Periotest value). In addition, it is defined that no mobility is confirmed when the Periotest value is between 0 and 9, mobility is sensed by palpation when the Periotest value is between 10 and 19, mobility is visually confirmed when the Periotest value is between 20 and 29, and teeth are moved by tongue and lip when the Periotest value is between 30 and 50. In the case of dental implants such as screws, the value of 10 or higher indicates insufficient osseointegration.

The results of measuring Periotest values are separately shown in Fig. 5(a) to (c) by weights of immediate loading. As shown in Fig. 5, immediately after implantation, the Periotest value of the titanium alloy screw was confirmed to be significantly lower than those of the other two screw types. This may be because the pitch width (0.5 mm) of the titanium alloy screw was shorter than the pitch width (0.8 mm) of the other two screw types.

Moreover, in all immediate loading groups, it was confirmed for all screws that Periotest values decreased from those immediately after implantation to seven and 28 days after implantation. This may be because new bone was formed around the screws and gradually became stable as time proceeded after implantation. In addition, it was confirmed for all screws that Periotest values seven and 28 days after implantation were lower in the case of 10g of immediate loading than the other cases.

As shown in Fig. 5, in all immediate loading groups, a decrease in Periotest value immediately after implantation of screw 1 of the present invention was confirmed to be more significant compared with the other two screw types. It was also confirmed that screw 1 of the present invention exhibited the lowest value of 10 or less 28 days after implantation in all immediate loading groups. This may be because more new bone sites were more quickly formed around screw 1 of the present invention compared with the other two screw types. Accordingly, it is considered that screw 1 of the present invention after implantation became stable more quickly than the other two screw types, maintaining good stability.

Next, screw 2 and screw 3 of the present invention were also similarly measured for mobility immediately after implantation and seven days after implantation. In addition, for comparison, a titanium alloy screw and a pure titanium screw were also measured. The results of measuring Periotest values are separately shown in Fig. 6(a) and (b) by weights of immediate loading. As shown in Fig. 6, immediately after implantation, all screws exhibited similar Periotest values; and seven days after implantation, all screws were confirmed to exhibit decreased Periotest values.

Moreover, as shown in Fig. 6(a), in cases of no immediate loading, Periotest values of screw 2 and screw 3 of the present invention significantly decreased to about 10, seven days after implantation, confirming significant differences when compared with those of the titanium alloy screw and the pure titanium screw. This may be because more new bone sites were quickly formed around screw 2 and screw 3 of the present invention than those formed around the other two screw types.

Moreover, as shown in Fig. 6(b), in cases of 10 g of immediate loading, the Periotest value of screw 3 of the present invention significantly decreased to about 10, seven days after implantation, confirming significant differences compared with the other three screw types. This may be because more new bone sites were quickly formed around screw 3 of the present invention than those formed around the other 3 screw types. In addition, screw 2 of the present invention exhibited a significant decrease in Periotest value seven days after implantation compared with the titanium alloy screw, but exhibited a Periotest value similar to that of the pure titanium screw.

Moreover, screw 2 and screw 3 of the present invention in the case of no immediate loading were also measured for mobility 28 days after implantation. The results are shown in Fig. 6(c). As shown in Fig. 6(c), screw 2 and screw 3 of the present invention quickly exhibited Periotest values equivalent to those 28 days after implantation, confirming that screw 2 and screw 3 became stable more quickly than screw 1 of the present invention shown in Fig. 5(a).

[Histological analysis]

Screw 1 of the present invention, a titanium alloy screw, and a pure titanium screw were histologically observed by screw type and by weights of immediate loading in order to evaluate the stability of the screws after implantation. Specifically, tissues surrounding the screws were observed seven and 28 days after implantation of the screws. In addition, regarding immediate loading, cases of no immediate loading and 50 g of immediate loading were observed. For observation, first, rats into which screws had been implanted were each subjected to perfusion fixation using a 4% paraformaldehyde solution. Both tibiae of lower extremities were excised, and then bone blocks each containing one screw were prepared. The bone blocks were fixed at 4°C in a 4% paraformaldehyde solution for 48 hours, and then washed for 30 minutes with running water. After washing, the bone blocks were dehydrated and delipidated with an ethanol rise system under ordinary temperature, followed by treatment with an intermediate agent, xylene. After treatment, resin filtration was performed in a resin permeate (Wako, "Osteoresin Embedding Kit") at 4°C, and then resin embedding was performed at 35°C. Resin blocks were processed with Saw Microtome Leica SP1600 (Leica Microsystems) into 100-$\mu$-thick resin sections, in parallel to the longitudinal direction of each screw. These sections were stained with Villanueva bone stain reagent (Polysciences, "Villanueva Osteochrome Bone Stain").

Stained sections were observed under an optical microscope, so as to find the ratio of the periphery of each screw to new bone (BIC; Bone-to-Implant Contact). Furthermore, the area ranging from the periphery of each screw to 240 $\mu$m(equals to the height of a screw blade) from the periphery was designated as an evaluation site for analysis (ROI; Region of Interest), and then the percentage of the area of new bone formed in ROI (BA; Bone Area) was found. BIC and BA were analyzed using ImageJ (National Institutes of Health).

In addition, BIC and BA are specifically found by the following formulae.

$$\text{BIC (\%)} = [\text{new bone mass (}\mu\text{m) in contact with screw surface/} \\ \text{Peripheral length of screw portion implanted (}\mu\text{m})] \times 100$$

$$\text{BA (\%)} = [\text{new bone area (}\mu\text{m}^2\text{) in ROI/} \\ \text{ROI area (}\mu\text{m}^2\text{)]} \times 100$$

Moreover, when the periphery of a screw tip portion was included in an analysis range, the range includes the existing cortical bone region more than necessary, and thus the resulting analytical value is inappropriate to represent new bone mass. Hence, the periphery of a screw tip portion was excluded from the analysis range.

Optical microscopic photographs of stained sections seven and 28 days after implantation of each screw are shown in Fig. 7. Moreover, BIC and BA found on the basis of optical microscopic photographs shown in Fig. 7 are shown in Fig. 8 and Fig. 9, respectively. In addition, the images of tissue sections seven days after implantation in Fig. 7(A), (B), (E), (F), (I), and (J) indicate osseointegration of cortical bone portions (subjected to implantation) with screws. It is thus considered that the repair of the existing cortical bone damaged by implantation of the screws was almost completed seven days after implantation.

As shown in Fig. 7(A) to (D), in the case of the titanium alloy screw, no clear new bone formation was observed seven days after implantation regardless of the presence or the absence of immediate loading, however, more sites of new bone formation were observed on the surfaces of screw blades subjected to 50 g of immediate loading 28 days after implantation (see white triangle arrows in Fig. 7, the same applies to the following). Moreover, as shown in Fig. 7(E) to (H), in the case of the pure titanium screw, significant new bone formation was not observed both seven and 28 days after implantation regardless of the presence or the absence of immediate loading. Moreover, as shown in Fig. 7(I) to (L), in the case of screw 1 of the present invention, more sites of new bone formation were observed on screw blades seven days after implantation, and particularly more sites of new bone formation were confirmed in the case of 50 g of immediate loading. Twenty eight days after implantation, sites of new bone formation were observed on the surfaces of screw blades, and more sites of new bone formation could be significantly confirmed in the case of particularly 50 g of immediate loading.

As shown in Fig. 7, in the case of screw 1 of the present invention, more sites of new bone formation can be confirmed than those formed around the other two screw types regardless of the presence or the absence of immediate loading. This may be because screw 1 of the present invention was better than the other two screw types in biocompatibility. In particular, more sites of new bone formation were observed in tissue sections surrounding screw 1 of the present invention seven days after implantation (see Fig. 7(I) and (J)) than those observed around the other 2 screw types. Hence, the amorphous alloy composition of screw 1 of the present invention can be said to be effective for new bone formation.

As shown in Fig. 8, the BIC of screw 1 of the present invention was significantly higher seven days after implantation than those of the other two screw types regardless of the presence or the absence of immediate loading. Moreover, 28 days after implantation, the BIC of screw 1 of the present invention was confirmed to be significantly higher than those of the other two screw types in the case of no immediate loading. Furthermore, as shown in Fig. 9, BA of screw 1 of the present invention was significantly higher seven days after implantation than those of the other two screw types in the case of no immediate loading. BA of screw 1 of the present invention subjected to 50 g of immediate loading was confirmed to be significantly higher than that of the pure titanium screw. Twenty eight days after implantation, BA of screw 1 of the present invention was confirmed to be significantly higher than those of the other two screw types regardless of the presence or the absence of immediate loading. These results revealed that after implantation, more sites of new bone were formed on the surface of screw 1 of the present invention than those formed on the other two screw types, and the new bone mass increased as days proceeded. Accordingly, it can be said that screw 1 of the present invention after implantation becomes stable more quickly than the other two screw types, maintaining good stability.

For evaluation of the stability of screw 2 and screw 3 of the present invention and a pure titanium screw after implantation, tissues surrounding the screws were observed seven days after implantation of screws by screw type (without immediate loading). A staining method employed for observation was the same method as in Fig. 7. Optical microscopic photographs of stained sections for each screw seven days after implantation are shown in Fig. 10.

As shown in Fig. 10(a) to (c), in the cases of screw 2 and screw 3 of the present invention, more sites of new bone formation were observed seven days after implantation than those formed around the pure titanium screw. This may be because screw 2 and screw 3 of the present invention are better than the pure titanium screw in biocompatibility. It can be said that the amorphous alloy compositions of screw 2 and screw 3 of the present invention are compositions effective for new bone formation. Furthermore, it can be said that screw 2 and screw 3 of the present invention after implantation become stable more quickly than the pure titanium screw, maintaining good stability.

[Dissolution test]

Amorphous alloys and pure titanium composing screws 1-3 of the present invention and a pure titanium screw, respectively, were tested for dissolution of components. In this test, first, 5.0 ml of a simulated body fluid (pH7.4) was added to a 15 ml of plastic tube, and then a metallic foil having the surface area of 384 mm$^2$ and formed of each metal was immersed in the fluid. These tubes were placed in a thermostatic bath and then maintained at 37°C for seven days. Subsequently, the amount of each metal ion of Al, V, Ti, Ni, Cu, and Zr dissolved in simulated body fluids was measured by ICP-MS (Inductively Coupled Plasma Mass Spectrometer). In addition, as "control", the amount of each metal ion was measured even in cases where no metallic foil was added.

Measurement results are shown in Fig. 11. As shown in Fig. 11, dissolved Ti levels were confirmed to be lower, but dissolved Cu and Zr levels were confirmed to be higher in the cases of amorphous alloys ("Zr70", "Zr68", and "Zr72" in Fig. 11) composing screws 1-3 of the present invention than in the case of pure titanium ("pure Ti" in Fig. 11) composing the pure titanium screw. They are natural results in view of each metal component. Moreover, the dissolved Ni level, a major cause of metal allergy, of the amorphous alloy composing each of screws 1-3 of the present invention, was low and almost the same as that of pure titanium composing the pure titanium screw, and no significant difference was observed. Accordingly, screws 1-3 of the present invention are considered to have no problem concerning allergies, similarly to pure titanium screws.

[Measurement of screw mobility using surface-treated material]

An orthodontic anchor screw (screw 1 of the present invention) made of an amorphous alloy having a composition of $Zr_{70}Ni_{16}Cu_6Al_8$ was heated at 350°C for one hour, so as to produce an orthodontic anchor screw made of surface-treated $Zr_{70}Ni_{16}Cu_6Al_8$ amorphous alloy (hereinafter, referred to as "surface-treated screw"), wherein the surface was coated with zirconia ceramics. The thus produced screw was tested for screw mobility using rats. For comparison, screw 1 of the present invention was subjected to the same test. Screw 1 of the present invention and the surface-treated screw used for the test are shown in Fig. 12(a) and (b), respectively.

The method for testing screw mobility employed herein was the same method as in Fig. 5 and Fig. 6. No immediate loading was performed. The results of measuring Periotest values are shown in Fig. 13. As shown in Fig. 13, seven days after implantation, the surface-treated screw exhibited the Periotest value significantly lower than that of screw 1 of the present invention. It can be said that the surface-treated screw had high stability. Furthermore, 28 days after implantation, the surface-treated screw and screw 1 of the present invention both exhibited decreases in Periotest value, indicating improved stability. However, there was no significant difference between the two. Accordingly, a Periotest value similar to that 28 days after implantation was obtained early in the case of the surface-treated screw, and it was thus confirmed that the surface-treated screw becomes stable more quickly than the screw 1 of the present invention.

[Cell adhesion test using surface-treated material]

A pure titanium foil (grade2; The Nilaco Corporation), a $Zr_{70}Ni_{16}CuAl_8$ amorphous alloy foil, and a surface-treated $Zr_{70}Ni_{16}Cu_6Al_8$ amorphous alloy foil having the surface coated with zirconia ceramics (prepared by heating a metallic glass foil at 350°C for one hour) were cut into a size of 6 x 6 mm. After sterilization, rat bone marrow cells were seeded, $2 \times 10^4$ cells each, and then cultured. To examine the cell adhesion ability of cells after 1 day of culture, actin (green), vinculin (red), and nuclei (blue) were stained by immunofluorescence staining, and then observed under a confocal microscope. Observation results are shown in Fig. 14.

As shown in Fig. 14, cell adhesion to the surfaces and cell growth were confirmed for all of the pure titanium foil, the $Zr_{70}Ni_{16}Cu_6Al_8$ amorphous alloy foil, and the surface-treated $Zr_{70}Ni_{16}Cu_6Al_8$ amorphous alloy foil. Accordingly, it can be said that the amorphous alloy and the surface-treated amorphous alloy to be used for the dental members of embodiments of the present invention have biocompatibility equivalent to or better than that of pure titanium.

As revealed by the above experimental results, the dental members of embodiments of the present invention quickly exhibit new bone formation around an area subjected to implantation and are excellent in stability of engraftment to bone after implantation, compared with titanium alloy screws and pure titanium screws. Moreover, the dental members of embodiments of the present invention exhibit high removal torques, so that the dental members do not fall off inadvertently after implantation, and can compensate for decreases in mechanical friction due to the decreased surface areas after size reduction. Accordingly, the dental members of embodiments of the present invention can be reduced in size smaller than conventional titanium alloy dental members and pure titanium dental members. For example, an orthodontic anchor screw or a dental implant in a screw shape wherein the core diameter of the screw part is as thin as 0.9 mm as shown in Fig. 15(a), and the same in a button shape wherein the length of the screw part is as short as about 2.5 mm as shown in Fig. 15(b) can be produced.

As described above, the dental members of embodiments of the present invention can be reduced in size smaller than conventional products, so that the dental members can be designed as orthodontical implants that do not damage tooth roots upon implantation, are completely safe for living bodies, and do not fall off. Furthermore, the design of short dental members as shown in Fig. 15(b) are employed, so that the dental members can be safely implanted into every sites of alveolar bone and palatine bone. In addition, screw heads shown in Fig. 15 are intended for orthodontic ligature wires, elastic rubber, or coil springs to be tied therewith or for wires to be embedded therein. Screw heads are not limited to these screw heads designed as shown in Fig. 15 and may be in any form, as long as they can exhibit these functions.

**Claims**

1. A dental member, containing an amorphous alloy having a composition represented by formula: $Zr_aNi_bCu_cAl_d$, wherein a, b, c, and d denote at.%, and "a" ranges from 67 to 73 at.%, "b" ranges from 11 to 17 at.%, "c" ranges from 5 to 13 at.%, and "d" ranges from 5 to 9 at.%.

2. The dental member according to claim 1, comprising an orthodontic anchor screw or a dental implant.

3. The dental member according to claim 1, comprising an orthodontic anchor screw wherein the screw part has a core diameter between 0.5 mm and 1.0 mm or a length between 2 mm and 5 mm.

4. The dental member according to claim 1, comprising a one-piece-type dental implant wherein the screw part has the largest diameter between 0.5 and 2.9 mm and a length between 2 and 13.4 mm, or a two-piece-type dental implant wherein the screw part has the largest diameter between 0.5 and 2.9 mm and a length between 2 and 5.9 mm.

5. The dental member according to any one of claims 1 to 4, wherein "c" ranges from 7 to 13 at.%, or, "d" ranges from 5 to 7 at.%.

6. The dental member according to any one of claims 1 to 4, wherein "a" ranges from 69 to 73 at.%, "b" ranges from 13 to 17 at.%, "c" ranges from 5 to 10 at.%, and "d" ranges from 5 to 9 at.%.


**Patentansprüche**

1. Zahnelement, das eine amorphe Legierung enthält, die eine Zusammensetzung aufweist, die durch die folgende Formel dargestellt wird: $Zr_aNi_bCu_cAl_d$, wobei a, b, c und d At.-% anzeigen, und "a" von 67 bis 73 At.-% reicht, "b" von 11 bis 17 At.-% reicht, "c" von 5 bis 13 At.-% reicht, und "d" von 5 bis 9 At.-% reicht.

2. Zahnelement nach Anspruch 1, umfassend eine orthodontische Ankerschraube oder ein Zahnimplantat.

3. Zahnelement nach Anspruch 1, umfassend eine orthodontische Ankerschraube, wobei das Schraubenteil einen Kerndurchmesser zwischen 0,5 mm und 1,0 mm oder eine Länge zwischen 2 mm und 5 mm aufweist.

4. Zahnelement nach Anspruch 1, umfassend ein einteiliges Zahnimplantat, wobei das Schraubenteil den größten Durchmesser zwischen 0,5 und 2,9 mm und eine Länge zwischen 2 und 13,4 mm aufweist, oder ein zweiteiliges Zahnimplantat, wobei das Schraubenteil den größten Durchmesser zwischen 0,5 und 2,9 mm und eine Länge zwischen 2 und 5,9 mm aufweist.

5. Zahnelement nach einem der Ansprüche 1 bis 4, wobei "c" von 7 bis 13 At.-% reicht, oder "d" von 5 bis 7 At.-% reicht.

6. Zahnelement nach einem der Ansprüche 1 bis 4, wobei "a" von 69 bis 73 At.-% reicht, "b" von 13 bis 17 At.-% reicht, "c" von 5 bis 10 At.-% reicht, und "d" von 5 bis 9 At.-% reicht.


**Revendications**

1. Elément dentaire, contenant un alliage amorphe présentant une composition représentée par la formule : $Zr_aNi_bCu_cAl_d$, dans laquelle a, b, c et d désignent un % at., et « a » vaut de 67 à 73 % at., « b » vaut de 11 à 17 % at., « c » vaut de 5 à 13 % at., et « d » vaut de 5 à 9 % at.

2. Elément dentaire selon la revendication 1, comprenant une vis d'ancrage orthodontique ou un implant dentaire.

3. Elément dentaire selon la revendication 1, comprenant une vis d'ancrage orthodontique dans laquelle la partie vis présente un diamètre de noyau compris entre 0,5 mm et 1,0 mm ou une longueur comprise entre 2 mm et 5 mm.

4. Elément dentaire selon la revendication 1, comprenant un implant dentaire du type une seule pièce dans lequel la partie vis présente le diamètre le plus grand compris entre 0,5 et 2,9 mm et une longueur comprise entre 2 et 13,4 mm, ou un implant de type deux pièces dans lequel la partie vis présente le diamètre le plus grand compris entre 0,5 et 2,9 mm et une longueur comprise entre 2 et 5,9 mm.

5. Elément dentaire selon l'une quelconque des revendications 1 à 4, « c » valant de 7 à 13 % at. ou « d » valant de 5 à 7 % at.

6. Elément dentaire selon l'une quelconque des revendications 1 à 4, « a » valant de 69 à 73 % at., « b » valant de 13 à 17 % at., « c » valant de 5 à 10 % at., et « d » valant de 5 à 9 % at.

Fig.1

(a)

(b)

(c)

(d)

Fig.2

(a)

(b)

Fig.3

Fig.4

(a)

(b)

Fig.5

Fig.6

Fig.7

A B C D

7 days after implantation
(No loading)

7 days after implantation
(50g of loading)

28 days after implantation
(No loading)

28 days after implantation
(50g of loading)

E F G H

7 days after implantation
(No loading)

7 days after implantation
(50g of loading)

28 days after implantation
(No loading)

28 days after implantation
(50g of loading)

I J K L

7 days after implantation
(No loading)

7 days after implantation
(50g of loading)

28 days after implantation
(No loading)

28 days after implantation
(50g of loading)

Fig.8

(a)

(b)

Fig.9

(a)

(b)

Fig.10

(a)　　　　　　　　(b)　　　　　　　　(c)

7 days after implantation

Fig.11

a: vs Control
b: vs Pure titanium
c: vs Zr68

Fig.12

（a）

X50    500µm

（b）

X50    500µm

Fig.13

Fig.14

Objective lens ×20

(a)

(b)

(c)

Bar scale=100μm

Fig.15

(a)

0.018 inch

(b)

1.0mm

1.0mm

6.0mm

0.5mm

< 0.9mm

1.0mm

1.0mm

2.0mm or less

0.5mm

1.8mm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009215610 A

- US 2004072124 A1

**Non-patent literature cited in the description**

- **TARIQ N H et al.** Effect of prior compression treatment on the deformation behavior of Zr based bulk metallic glass. *MATERIALS CHEMISTRY AND PHYSICS,* 14 February 2014, vol. 143 (3), 1384-1390

- **ASAHI KAWASHIMA et al.** The corrosion behaviour of Zr-based bulk metallic glasses in 0.5M NaCl solution. *CORROSION SCIENCE,* 05 May 2011, vol. 53 (9), 2778-2784